# EUROPÄISCHE PATENTSCHRIFT

(11) **EP 3 572 120 B1**
(45) Veröffentlichungstag und Bekanntmachung des Hinweises auf die Patenterteilung: **22.05.2024**
(21) Anmeldenummer: 19185354.8
(22) Anmeldetag: 22.01.2016
(51) Int. Cl.: A61M 39/22, A61M 60/546, A61M 60/523, A61M 60/438, B03C 1/28, A61M 60/279, F16K 31/06, A61M 60/13, A61M 60/148

(54) **KATHETERVORRICHTUNG, ENTHALTEND EIN VENTIL ZUR STEUERUNG EINES FLUIDFLUSSES DURCH EINEN KATHETER**
CATHETER DEVICE, COMPRISING A VALVE FOR CONTROLLING A FLOW OF A FLUID THROUGH A CATHETER
DISPOSITIF DE CATHÉTER COMPORTANT UNE SOUPAPE DE COMMANDE D'UN ÉCOULEMENT DE FLUIDE À TRAVERS UN CATHÉTER

(30) Priorität: 22.01.2015 EP 15152201; 22.01.2015 EP 15152205
(43) Veröffentlichungstag der Anmeldung: 27.11.2019
(62) Teilanmeldung aus: 16701465.3
(73) Patentinhaber: ECP Entwicklungsgesellschaft mbH, 52074 Aachen (DE)
(72) Erfinder: LIEBING, Reiner, 13187 Berlin (DE)
(74) Vertreter: Pfenning, Meinig & Partner mbB

(56) Entgegenhaltungen:
- EP-A1- 2 246 078
- WO-A1-2008/101352
- DE-A1- 4 123 091
- US-A- 3 570 807
- US-A1- 2004 055 652
- US-A1- 2010 331 753
- US-A1- 2012 145 641
- US-A1- 2014 155 815
- US-A1- 2014 261 717

## Beschreibung

Die Erfindung liegt auf dem Gebiet der Mechanik und ist mit besonderem Vorteil auf dem Gebiet der Medizintechnik anwendbar. Sie befasst sich insbesondere mit einem Ventil, das auch bei unterschiedlichen Druckverhältnissen ein sicheres Sperren eines Fluidflusses durch einen Katheter erlaubt. Die Erfindung beschreibt entsprechend eine Kathetervorrichtung welche im unabhängigen Anspruch 1 definiert ist.

Aus dem Stand der Technik sind zur Steuerung von Fluidflüssen vielfältige Absperrventile bekannt. Beispielsweise wird in der DE 20 2013 104711 A1 ein industriell entwickeltes Einweg-Absperrventil gezeigt. Ein einfaches Rückschlagventil ist beispielsweise aus der DE 112009 003 676 T5 bekannt. Derartige Rückschlagventile werden beispielsweise in der Kraftfahrzeugtechnik angewendet. Solche Absperrventile haben grundsätzlich einen eng umrissenen Anwendungsbereich, wobei Probleme entweder bei Über- oder Unterdruck oder bei Druckschwankungen auftreten, da solche Ventile oft nur für einen engen Druckbereich konzipiert sind. Für eine Anwendung im medizinischen Bereich, jedoch auch in anderen speziellen Bereichen, ist oft zudem eine Medientrennung unerlässlich, das heißt, der eigentliche Fluidkanal einschließlich der Sperrstelle muss von anderen Elementen eines Ventils, wie beispielsweise den Antriebselementen, hermetisch abgeschlossen sein.

Zudem ist für Anwendungen im Medizintechnikbereich eine einfache Reinigungs- und Sterilisationsmöglichkeit oder wahlweise eine einfache Austauschbarkeit wichtig, wenn Einwegbauteile verwendet werden.

Die Druckschrift WO 2008/101352 A1 zeigt eine wiederverwendbare Magnetvorrichtung zum Absaugen von Eisenpartikeln aus einem Fluidkörper, wobei die Vorrichtung mehrere Magnete und weiche Eisenmetallabstandshalter umfasst, die in abwechselnder Reihenfolge angeordnet sind, um einen Stapel zu bilden, wobei benachbarte Magnete mit gleichen Polen gegenüberliegend angeordnet sind.

Die US 2010/331753 A1 zeigt ein Verfahren zur Behandlung von Malaria, wobei die Behandlung den Schritt der Entfernung von mit Malaria infizierten roten Blutkörperchen aus dem Blut des Patienten umfasst. Das Blut wird aus dem Kreislauf des Patienten entnommen und durch ein Blutreinigungsgerät geleitet, das die infizierten roten Blutkörperchen selektiv von allen anderen Blutbestandteilen entfernt und das gereinigte Blut wieder in den Kreislauf des Patienten zurückführt. Eine Blutreinigungsvorrichtung, die zur Durchführung der therapeutischen Verfahren der Erfindung nützlich ist, wird ebenfalls beschrieben. Die Vorrichtung nutzt die magnetischen Eigenschaften des in den infizierten roten Blutkörperchen enthaltenen Hämozoins und umfasst eine oder mehrere Trennkammern, durch die Blut durch ein Magnetfeld mit hohem Gradienten fließt, das durch eine Anordnung von Drähten erzeugt wird, die von den Kammern getrennt sind und nicht in Kontakt mit dem Patientenblut stehen.

Die EP 2 246 078 A1 zeigt eine Anordnung mit einer Welle, die sich in einem mit Fluid gefüllten Gehäuse erstreckt und von einem Antrieb, der außerhalb des Gehäuses angetrieben wird, betätigt wird. Die Welle weist eine spindelförmige Umrisskontur einer Außenumfangsfläche und eine Oberflächenstruktur auf, die das Fluid in einer Strömungsrichtung in einer Längsrichtung der Welle während der Drehung befördert.

US 2004/055652 A1 betrifft eine Dosiskontrollvorrichtung. Die Vorrichtung weist zwei Anker auf, die durch mindestens eine Feder gegen einen Ventilsitz gedrückt werden. Eine Spule induziert ein Magnetfeld, das die Anker gegen die Kraft der Feder antreibt und dadurch das Ventil öffnet. Die Anker können sich entlang einer gemeinsamen Achse in entgegengesetzte Richtungen bewegen. Die Vorrichtung kann auch einen Kern aufweisen, der zwischen den Ankern und einem Gehäuse um die Spule angeordnet ist. Der Kern und das Gehäuse leiten das Magnetfeld und reduzieren den Energiebedarf für die Erzeugung des Feldes. Der Strom kann in der Spule periodisch umgekehrt werden, um ein Entmagnetisierungsfeld bereitzustellen. Zusätzlich kann von der Spule ein Signal erzeugt werden, wenn ein extern angelegtes Magnetfeld, wie beispielsweise ein MRI, vorhanden ist. Ein entgegengesetztes Magnetfeld kann durch die Spule erzeugt werden oder der an die Spule gelieferte Strom kann eingestellt werden

In der US 2014/261717 A1 wird ein Ventilsystem bereitgestellt, das ein elektromechanisches Solenoid, ein Gasventil mit einer Magnetklappe, eine Stromquelle in elektrischer Verbindung mit dem elektromechanischen Solenoid und eine Steuerung umfasst. Die Steuerung ist so konfiguriert, dass sie die Stromquelle so steuert, dass sie eine Zwischenspannung liefert, die bewirkt, dass sich das elektromechanische Solenoid in eine neutrale Position zwischen einer Standardposition und einer voll betätigten Position bewegt. Auf diese Weise wird die Magnetklappe in einer neutralen Position zwischen zwei Gasöffnungen gehalten und ein Gasstrom zwischen den Öffnungen kann verwendet werden, um Fremdpartikel von der Magnetklappe zu entfernen.

Die DE 41 23 091 A1 beschreibt ein Ventil, insbesondere für eine Infusionspumpe, mit einem eine Kammer ausbildenden Gehäuse, in dessen Stirnwand eine erste Zuflussbohrung und eine erste Abflussbohrung und in dessen andere Stirnwand eine zweite Zuflussborhung und eine zweite Abflussbohrung eingebracht sind. Weiterhin umfasst das Ventil eine sich parallel zu der einen Stirnwand erstreckende erste Membran und eine sich parallel zu der anderen Stirnwand erstreckende zweite Membran sowie einen von der Kammer beweglich aufgenommenen Kolben aus einem permanentmagntischen Material, dessen Länge geringer als der Abstand zwischen den beiden Stirnwänden ist und eine in der Kammer um den Kolben umlaufend angeordnete erste Spule. Der Kolben wird bei Erregung der Spule wahlweise in Richtung auf die eine Stirnwand oder aber die andere Stirnwand in eine erste Position, in der die erste Membran die erste Zuflussbohrung und/oder die erste Abflussbohrung verschließt, oder aber in eine zweite Position, in der die zweite Membran die zweite Zuflussbohrung und/oder die zweite Abflussbohrung verschließt verlagert, und bei Nicht-Erregung der Spule aufgrund der Spannkraft der Membranen in eine Zwischenposition, in der die erste Zuflussbohrung mit der ersten Abflussbohrung und die zweite Zuflussbohrung mit der zweiten Abflussbohrung kommuniziert.

Die US 3 570 807 A zeigt ein elektromechanisches Steuerventil, bei dem ein Ventilteller magnetisch gegen einen Ventilsitz vorgespannt ist und bei Abnahme des magnetischen Moments mechanisch entsperrt wird. Eine Aufhängeplatte trägt den Ventilkegel auf der einen Seite und einen Anker auf der anderen Seite, um eine integrierte Baugruppe zu bilden. Ein Permanentmagnet zieht den Anker an, um dadurch den Ventilkegel gegen den Sitz zu drücken und die Aufhängungsplatte zu verformen. Eine Spule ist auf dem Anker angeordnet und entwickelt unter Spannung ein Magnetpotential im Gegensatz zum Magnetpotential des Permanentmagneten, um dadurch die Anziehungskraft des Permanentmagneten zu verringern, wodurch die verformte Aufhängungsplatte, die als Feder wirkt, den Ventilteller aus dem Sitz drückt.

Die US 2014/155815 A1 zeigt eine Wiederherstellungskatheter-Baugruppe mit einem Betätigungselement und einer mechanisch radial ausdehnbaren und zusammenziehbaren Wiederherstellungsvorrichtung, die betriebsmäßig mit dem Betätigungselement verbunden ist. Die Wiederherstellungsvorrichtung hat einen proximalen und einen distalen Blockierabschnitt und einen dazwischen liegenden zentralen Abschnitt. Die Wiederherstellungsvorrichtung kann durch Betätigung des Betätigungselements zumindest teilweise in eine erste, radial kollabierte Konfiguration und in eine zweite, radial expandierte Konfiguration gebracht werden. In der zweiten, radial expandierten Konfiguration haben der proximale und der distale Blockierabschnitt radiale Abmessungen, die größer sind als die radiale Abmessung des zentralen Abschnitts, wodurch zumindest teilweise eine Sammelkammer am zentralen Abschnitt definiert wird.

Die US 2012/145641 A1 lehrt, dass sich im Alter unerwünschte anergische zytotoxische T-Zellen ansammeln. Diese Zellen haben oft virus-spezifische T-Zellen-Rezeptoren sowie andere Oberflächenmarker, die sie von ihren jugendlichen Gegenstücken unterscheiden, und es wird angenommen, dass sie eine wichtige Rolle bei der Abnahme des Immunsystems mit dem Alter spielen. Die US 2012/145641 A1 offenbart die Verwendung von magnetischen Zellseparationsmethoden und -apparaten, um seneszente T-Zellen zu entfernen, die durch die Oberflächenmarker CD8+, KLRG1+ und CD8+, CD28- definiert sind.

Der vorliegenden Erfindung liegt vor dem Hintergrund des Standes der Technik somit die Aufgabe zugrunde, eine Kathetervorrichtung bzw. ein Ventil zu schaffen, das bei geringen Fluiddurchsätzen auch unter unterschiedlichen Druckverhältnissen ein sicheres Absperren und Öffnen eines Fluidkanals erlaubt.

Die Aufgabe wird mit den Merkmalen der Erfindung gemäß den Patentansprüchen gelöst. In den Unteransprüchen sind vorteilhafte Ausgestaltungen der Erfindung angegeben.

Dies betrifft zunächst eine Kathetervorrichtung, enthaltend einen Katheter zum Einführen in ein Lebewesen sowie mindestens ein Lumen zum Führen eines Fluidflusses innerhalb eines Teils der Kathetervorrichtung sowie enthaltend ein Ventil zur Steuerung eines Fluidflusses insbesondere durch einen Katheter, mit einem Ventilsteuerraum, in dem ein Zuflusskanal mit einer Zuflussöffnung und ein Abflusskanal mit einer Abflussöffnung münden, und mit einem in dem Ventilsteuerraum gesteuert bewegbaren Verschlusselement, das in wenigstens einer ersten Stellung die Abflussöffnung, in wenigstens einer zweiten Stellung die Zuflussöffnung verschließt und das in wenigstens einer dritten Stellung einen Verbindungskanal zwischen der Zuflussöffnung und der Abflussöffnung offenhält, wobei ein Ventilantrieb vorgesehen ist, der das Verschlusselement wahlweise wenigstens in die erste, zweite oder dritte Stellung bewegt, wobei das mindestens eine Lumen mit dem Zuflusskanal oder dem Abflusskanal fluidleitend verbunden ist.

Wie weiter unten noch einmal näher ausgeführt wird, ist mit der Kathetervorrichtung eine sehr genaue Führung von Spülflüssigkeit, sowohl in Hinblick auf die Spülrichtung als auch in Hinblick auf die Spülvolumina und Spülflüsse in den Kathetern möglich. Dies geschieht insbesondere vor dem Hintergrund von biegsamen drehbaren Wellen, die besondere Sogwirkung bzw. wechselnde Druckverhältnisse, je nach Länge und Einbauort, aufweisen. Im Übrigen ist gerade auf diesem Gebiet der Medizintechnik eine einwandfreie Funktion der Ventile von größter Wichtigkeit. Insbesondere ist auch die Kopplung mit einer Separiereinrichtung zum Entfernen von Verschmutzungen und Abrieb, beispielsweise Metallabrieb, aus der Spülflüssigkeit steuerbar. Dies kann beispielsweise der Fall sein, wenn bei einem "Durchspülen" einer Separiereinrichtung (also einer Einrichtung zum Auffangen/Einfangen von Abrieb etc.) eine temporäre Richtungsumkehr zur Reinigung sinnvoll ist.

Beispiele für eine derartige Separiereinrichtung sind beispielsweise in der parallelen, tagesgleich eingereichten ECP 46 PCT (WO 2016/116630) der ECP GmbH erläutert. Desweiteren wird die Priorität der beiden Voranmeldungen EP 15152201.8 und EP 15152205.9 beansprucht.

Eine Ausführungsform sieht vor, dass der Katheter eine drehbare Welle aufweist. Diese ist beispielsweise flexibel, insbesondere derart flexibel, dass sie an eine Krümmung eines menschlichen Aortenbogens anpassbar ist, also dass beispielsweise bei einem Einführen einer entsprechenden Welle in die Fernoralarterie und einem Weiterführen entlang der Aorta ein Pumpenkopf an der Spitze der flexiblen Welle in den Ventrikel des Herzens eingeführt wird und sich bei einem Schieben des Katheters in Richtung der aufsteigenden Aorta der Katheter selbsttätig der Krümmung des Aortenbogens anpasst.

In einer Ausführungsform ist die Antriebseinrichtung außerhalb des Lebewesens (also außerhalb der Fernoralarterie im obigen Beispiel) gewählt, die drehbare Welle läuft in die Fernoralarterie hinein bis beispielsweise in den linken Ventrikel zum Antrieb eines dort positionierten Pumpenkopfes einer Katheterpumpe (Herzpumpe).

Eine weitere Ausführungsform sieht vor, dass der Katheter mehr als ein Lumen aufweist, wobei mindestens ein Lumen für die Fluidführung in distaler Richtung und mindestens ein Lumen für die Fluidführung in proximaler Richtung ausgeführt ist.

In einer Ausführungsform können zwischen 10 und 90 Prozent des in distaler Richtung geführten, in einem ersten Lumen geführten Fluidstroms in einem anderen Lumen in proximaler Richtung rückgeführt werden. Bei der sogenannten "Y-Spülung" wird also ein Fluidstrom in distaler Richtung gefördert, ein Teil der Spülflüssigkeit gelangt beispielsweise in das Herz am distalen Ende des Katheters, ein anderer Teil der Spülflüssigkeit tritt durch das jeweils andere Lumen dann wieder aus. Es sind auch noch komplexere Ausführungsformen möglich, bei denen zusätzlich auch noch die Antriebseinrichtung (d. h. der Antrieb, der beispielsweise außerhalb des Körpers befindlich ist) mit einem Fluidstrom versorgt wird, um beispielsweise Abrieb im Bereich der Lager etc. aus der Kathetervorrichtung zu entfernen.

Daher sehen Ausführungsformen auch vor, dass ein, zwei, drei oder auch mehr Ventile vorgesehen sind, um beispielsweise komplexere Spülvorgänge bzw. bewusste Umkehrungen der Spülrichtung (beispielsweise zur Reinigung einer Separiereinrichtung) durchzuführen.

Eine Ausführungsform sieht vor, dass mehrere Ventile, beispielsweise zwei Ventile, vorgesehen sind, wobei der Abfluss des ersten Ventils mit einem Zufluss zur Antriebseinrichtung der Kathetervorrichtung verbunden ist und ein zweites Ventil vorhanden ist, wobei der Zufluss des zweiten Ventils mit einem in proximaler Richtung fluidführenden Lumen des Katheters verbunden ist.

Verschiedene Ausführungsformen der Ventile in einer Kathetervorrichtung 100 werden im speziellen Beschreibungsteil gezeigt. Dies betrifft beispielsweise die Ausführungsformen 8, 9, 10, 11 und 12.

Es sei außerdem erwähnt, dass die Ventile so ausgeführt sind, dass ein Ventilantrieb des Verschlusselementes separierbar ist, d. h. werkzeugfrei trennbar von dem Rest der Anordnung, vor allem um einen leichteren Austausch zu ermöglichen und die Sterilität zu geringen Kosten auch bei einer Mehrfachverwendung von Teilen der Anordnung sicherzustellen.

Es sei außerdem ergänzt, dass der Ventilantrieb auf beliebige Weisen erfolgen kann. Neben magnetisch wirkenden Antrieben kann der Antrieb auch rein mechanisch erfolgen, er kann elektrisch erfolgen oder auch induktiv; hier sind jegliche Antriebe möglich, die es einem Fachmann erlauben, eine Bewegung eines Verschlusselementes in die erste, zweite und/oder dritte Stellung zu erreichen.

Die Anmeldung bezieht sich unter anderem auf ein Ventil zur Steuerung eines Fluidflusses durch einen Katheter, mit einem Ventilsteuerraum, in dem ein Zuflusskanal mit einer Zuflussöffnung und ein Abflusskanal mit einer Abflussöffnung münden, und mit einem in dem Ventilsteuerraum gesteuert bewegbaren Verschlusselement, das in wenigstens einer ersten Stellung die Abflussöffnung, in wenigstens einer zweiten Stellung die Zuflussöffnung verschließt und das in wenigstens einer dritten Stellung einen Verbindungskanal zwischen der Zuflussöffnung und der Abflussöffnung offenhält, wobei ein Ventilantrieb vorgesehen ist, der das Verschlusselement wahlweise wenigstens in die erste, zweite oder dritte Stellung bewegt. In der dritten oder weiteren Ventilstellungen können in einer Ausgestaltung verschiedene Ventilspaltgrößen zur Steuerung eines Durchflusses ansteuerbar sein.

Durch die Konstruktion kann das Ventil den Fluiddurchfluss sowohl durch Verschließen der Abflussöffnung als auch Verschließen der Zuflussöffnung sperren. Die Begriffe Zuflusskanal und Abflusskanal sind so gewählt, dass der Zuflusskanal mit seiner Mündung den Kanal bezeichnet, der unter normalen oder statistisch häufigsten Bedingungen und Druckverhältnissen als Zuflusskanal fungiert. Analog verhält es sich mit dem Begriff Abflusskanal.

Damit ergibt sich sowohl bei einem höheren Druck im Zuflusskanal als im Abflusskanal als auch bei einem höheren Druck im Abflusskanal als im Zuflusskanal jeweils die Möglichkeit, durch den Druckunterschied die Schließkräfte und damit den dichten Sitz des Verschlusskörpers zu unterstützen, indem bei einem Überdruck im Zuflusskanal der Verschlusskörper die Abflussöffnung verschließt, während bei einem Überdruck im Abflusskanal der Verschlusskörper die Zuflussöffnung verschließt. In jedem dieser Fälle wird durch den Druckunterschied zwischen dem Ventilsteuerraum und dem jeweils verschlossenen Kanal der Verschlusskörper zusätzlich zu den mechanischen Antriebskräften in der Verschlussstellung gehalten.

Da der Zuflusskanal in bestimmten Fällen als Abflusskanal fungieren kann und umgekehrt, könnte auch einfach der Zuflusskanal als erster Kanal und der Abflusskanal als zweiter Kanal bezeichnet werden.

Die zuverlässige Dichtung ist insbesondere in solchen System wichtig, in denen wechselnde oder sich langfristig ändernde Druckverhältnisse herrschen können. Solche Verhältnisse herrschen beispielsweise in Kathetern, die zur Führung von mechanisch antreibbaren rotierenden Wellen und/oder zum Spülen derartiger Katheter verwendet werden. Üblicherweise wird beim Spülen von Kathetern, die eine rotierende Welle führen, ein sehr geringer Flüssigkeitsdurchsatz angestrebt, der unter anderem dazu führt, dass Abriebpartikel der Welle nur in eine definierte Richtung weiterbewegt werden. Derartige rotierende Wellen werden oft aus einem Bündel verseilter Drähte hergestellt, wobei das Bündel an seiner Außenkontur eine Wendelform aufweist. Diese Wendelform bewirkt bei schneller Rotation der Welle eine Förderwirkung der die Welle umgebenden Spülflüssigkeit, so dass zu der eigentlichen Umlaufbewegung der Spülflüssigkeit, die durch eine Spülmittelpumpe bewirkt wird, eine zusätzliche Sogwirkung hinzutritt. Diese Sogwirkung ist zeitlich veränderlich, da die Kontur der Welle sich durch Abnutzung und Abrieb mit der Zeit verändert. Hierdurch treten in entsprechenden Spülkathetern auch veränderliche Druckverhältnisse auf, die bis zu einer Umkehrung des Spülmittelflusses führen können. Dabei wird bei einem erfindungsgemäßen Ventil angestrebt, die Möglichkeit einer sicheren Sperrung/Steuerung eines derartigen Fluidflusses unabhängig von den Druckverhältnissen zu erreichen. Wie oben ausgeführt, ergibt sich dabei die Möglichkeit, je nach Druckgefälle einen Verschluss des entsprechenden Fluidkanals durch wahlweises Verschließen der Zuflussöffnung oder der Abschlussöffnung (oder: erste Öffnung oder zweite Öffnung) zu erreichen, wobei die jeweils verschlossene Öffnung danach ausgewählt werden kann, in welcher Position die Verschlussstellung des Verschlusselementes an der Öffnung durch das Druckgefälle stabilisiert wird.

Ausgestaltungen sehen vor, dass zumindest Teile des Ventils als Einwegbauteile konzipiert sind. Dies ist beispielsweise für den Bereich der Medizintechnik sinnvoll. Auf diese Weise können fluidleitende Bauteile (beispielsweise der Ventilsteuerraum) als Einwegbauteile austauschbar sein. Kostenträchtige Bauteile, wie z. B. der Ventilantrieb des Verschlusselements, die vorzugsweise nicht unmittelbar mit dem Fluid in Berührung kommen, sind als wiederverwertbare Bauteile vorgesehen. So ist es beispielsweise möglich, dass der Ventilsteuerraum Teil eines Katheters, insbesondere eines Katheterschlauches aus beispielsweise Kunststoffmaterial, ist. Als Ventilantriebe kommen beispielsweise Systeme in Betracht, die berührungsfrei (z. B, magnetisch, induktiv) arbeiten oder elastische Eigenschaften z. B. des Ventilsteuerraums ausnutzen, um Antriebskräfte zu übertragen.

Eine Ausgestaltung sieht vor, dass die dritte Stellung des Verschlusselementes zwischen der ersten und der zweiten Stellung liegt.

Dies bewirkt, dass aus jeder der Verschlussstellungen durch eine minimale Bewegung des Verschlusselementes ein Freimachen des Fluidkanals ermöglicht ist und dass aus der dritten Stellung jede der beiden Verschlussstellungen schnell, sicher und durch eine minimale Bewegung des Verschlusselementes erreichbar ist.

Weiter kann vorgesehen sein, dass der Ventilsteuerraum mit Ausnahme der Zuflussöffnung und der Abflussöffnung allseits fluiddicht abgeschlossen ist.

Diese Konstruktion bewirkt eine vollständige Medientrennung, so dass die Elemente des Ventilantriebs nicht mit dem eigentlich zu steuernden Fluid in Verbindung kommen.

Beispielsweise kann dies dadurch erreicht werden, dass das Verschlusselement eine bewegliche Membran aufweist, die den Ventilsteuerraum fluiddicht abschließt und derart auslenkbar ist, dass wahlweise die Zuflussöffnung oder die Abflussöffnung durch Teile der Membran verschließbar sind.

Im unausgelenkten Zustand bildet die Membran, die umlaufend fluiddicht mit den übrigen Teilen des Ventilsteuerraums verbunden, insbesondere verklebt oder verschweißt ist, einen Abschluss des Ventilsteuerraums. Das zu steuernde Fluid kann an der Membran vorbei zwischen der Zuflussöffnung und der Abflussöffnung oder umgekehrt strömen. Die Membran ist dabei elastisch oder plastisch verformbar ausgeführt, so dass sie auslenkbar ist, und zwar so weit, dass sie oder ein Teil von ihr wahlweise vor eine der beiden Öffnungen, die Zuflussöffnung oder die Abflussöffnung, bringbar und gegen diese Öffnung pressbar ist. Hierdurch wird ein Abschließen der Zuflussöffnung oder der Abflussöffnung erreicht. Zum Öffnen der jeweiligen Zufluss- oder Abflussöffnung wird die Membran entspannt, so dass sie sich im Idealfall von selbst oder durch ihre Eigenspannung in ihre Ausgangslage bewegt.

Eine vorteilhafte Ausgestaltung der Erfindung sieht hierzu vor, dass ein Antriebshebel des Ventilsantriebs die Membran wenigstens in der ersten und zweiten Stellung auslenkt.

Der Antriebshebel greift somit unter die Membran und lenkt diese so weit aus, dass sie zwischen der Zuflussöffnung bzw. der Abflussöffnung und dem Antriebshebel eingeklemmt wird und die jeweilige Öffnung verschließt. Wird der Antriebshebel des Ventilantriebs zurückbewegt, so löst sich die Membran von der jeweiligen Öffnung wieder.

Der Antriebshebel kann dabei an seinem gegen die Membran drückenden Ende beispielsweise eine Kugel- oder Ellipsoidform aufweisen, die besonders gut zum Verschluss einer Öffnung im Ventilsteuerraum unter Zwischenlage der Membran geeignet ist.

Eine weitere vorteilhafte Ausgestaltung der Erfindung kann vorsehen, dass das Verschlusselement durch einen magnetisch wirkenden Ventilantrieb antreibbar ist.

Beispielsweise durch eine derartige Gestaltung des Ventilantriebs wird es möglich, das Ventil selbst von der Antriebseinheit vollständig zu trennen, beispielsweise dadurch, dass der Ventilsteuerraum von dem magnetischen Antrieb durch eine gas- oder fluidundurchlässige Wand getrennt ist. Auch der gesamte den Ventilsteuerraum umgebende Ventilkörper kann nochmals durch eine zusätzliche medientrennende Wand von den Elementen getrennt sein, die die Magnetfelder für den Antrieb erzeugen.

Beispielsweise kann der vom Ventilsteuerraum abgewandte Teil des Antriebshebels magnetisch ausgestaltet und durch einen externen Magneten auslenkbar sein.

Die Erfindung kann zudem vorteilhaft dadurch ausgestaltet werden, dass das in dem Ventilsteuerraum angeordnete Verschlusselement magnetisch aktiv ist und mit einem Magnetfeld des Ventilantriebs wechselwirkt.

In diesem Fall kann das Verschlusselement oder ein Teil des Verschlusselementes aus einem Magnetkörper bestehen, der beispielsweise magnetisiert sein kann oder der zumindest aus einem ferromagnetischen Stoff bestehen kann und der im Feld eines externen Magneten antreibbar ist. In diesem Fall kann der magnetisch aktive Teil des Verschlusselementes mit einer nicht magnetisch aktiven fluidundurchlässigen Schicht abgedeckt sein, so dass das Fluid, dessen Durchfluss durch das Ventil gesteuert werden soll, mit dem magnetisch aktiven Teil nicht in Berührung kommt.

Eine weitere vorteilhafte Ausgestaltung sieht vor, dass sowohl der Ventilsteuerraum als auch die mit dem Verschlusselement mechanisch verbundenen Teile des Ventilantriebs mit Ausnahme der Zufluss- und Abflussöffnung fluiddicht abgeschlossen und insbesondere von einer Magnetfelderzeugungseinrichtung des Ventilantriebs separierbar sind.

Durch diese Ausgestaltung ist es beispielsweise mit geringem Aufwand möglich, einen Teil des Ventils, der den Ventilsteuerraum und ggf. einen Verschlusskörper oder Teile des Verschlusskörpers enthält, von der Magnetfelderzeugungseinrichtung zu separieren (das heißt vorzugsweise zu trennen / zerstörungsfrei abzukoppeln) und als Einweg-Bauteil zu ersetzen. Die Magnetfelderzeugungseinrichtung kann ihrerseits dann mehrfach verwendet werden.

Eine weitere vorteilhafte Ausgestaltung der Erfindung sieht vor, dass das Verschlusselement durch ein elastisches Federelement vorzugsweise in die dritte Stellung bewegt wird.

Das Verschlusselement kann durch das elastische Federelement, beispielsweise eine Schraubenfeder, in der dritten Stellung gehalten und gegen die Kraft der Feder durch einen Antrieb in die erste oder zweite Stellung gebracht werden. Nach Abschalten des Ventilantriebs ist vorgesehen, dass das elastische Federelement das Verschlusselement selbstständig wieder in die dritte Stellung bewegt. Auf diese Weise ist erreicht, dass bei einem Ausfall der elektrischen Versorgung, sofern Elektromagnete für den Antrieb verwendet werden, das Verschlusselement keiner äußeren Krafteinwirkung unterliegt und somit das Ventil im geöffneten Zustand stehen bleibt, Zudem wird durch das elastische Federelement das Lösen des Verschlusselementes aus der ersten Stellung und der zweiten Stellung unterstützt.

Die Erfindung kann zudem vorteilhaft dadurch ausgestaltet werden, dass unmittelbar am Ventilsteuerraum, insbesondere im Inneren des Verschlusselements ein Magnet als Teil einer Separiereinrichtung vorgesehen ist. In diesem Fall können im Ventilsteuerraum magnetische und magnetisierbare Partikel von dem Magnet der Separiereinrichtung gebunden werden, so dass sie von den Dichtflächen des Ventils ferngehalten werden. Dabei kann insbesondere vorgesehen sein, dass der/die Magnet(e) von Antriebsankern des einen Ventilantriebs separat, insbesondere beabstandet, vorgesehen sind.

Es kann allerdings auch vorgesehen sein, dass der Magnet der Separiereinrichtung mit einem oder mehreren Magneten des Ventilantriebs kombiniert oder verbunden ist, oder eine erste Funktionsfläche eines Magneten kann der Separierung von Partikeln dienen, während andere Funktionsflächen der Ventilfunktion dienen.

Die Erfindung kann sich zudem auf eine Schutzeinrichtung für ein Ventil beziehen, das mit einem strömenden Fluid in Verbindung steht, dadurch gekennzeichnet, dass entlang eines Strömungskanals für das Fluid, insbesondere eines Katheters, von dem Ventil beabstandet und insbesondere von diesem separiert eine Separiereinrichtung zur Rückhaltung von im Fluid befindlichen Partikeln mit wenigstens einem Magnetelement vorgesehen ist.

Die Separiereinrichtung kann vorteilhaft bezüglich der vorherrschenden Strömungsrichtung des Fluids stromaufwärts von dem Ventil vorgesehen sein, jedoch können die beiden genannten Elemente auch einfach hintereinander, insbesondere voneinander beabstandet, beispielsweise auch baulich voneinander getrennt, zum Beispiel in Form von zwei separaten Bauelementen mit verschiedenen Gehäusen, vorgesehen sein.

Das Ventil kann frei von magnetischen oder magnetisch wirkenden Elementen sein und beispielsweise insgesamt unmagnetisch sein. Es kann eine Dichtfläche aufweisen, die vor Partikeln geschützt werden soll.

Das Ventil kann auch magnetische Bauteile enthalten, wie beispielsweise einen Antriebsmagneten oder einen Anker. Das Magnetelement der Separiereinrichtung kann ein von den magnetischen Bauteilen des Ventils getrennter Magnet sein oder eine Funktionsfläche eines magnetischen Bauelementes, die ausschließlich die Funktion der Partikelseparation hat, wobei andere Funktionsflächen des magnetischen Bauelementes andere Funktionen des Ventils wie beispielsweise eine Antriebsfunktion ausüben können. In diesem letztgenannten Fall kann das Magnetelement der Separiereinrichtung mit einem magnetischen Bauelement des Ventils kombiniert, mit diesem zusammengefügt, zusammengefasst und insbesondere auch in einem Gehäuse zusammengefasst sein.

Die Funktionsfläche der Separiereinrichtung kann somit Partikel, insbesondere magnetische und/oder magnetisierbare Partikel einfangen und binden, bevor sie zu dem Ventil gelangen und somit die Ventilfunktion, beispielweise die Dichtfunktion der Dichtflächen, beeinträchtigen können.

Im Folgenden wird die Erfindung anhand von Ausführungsbeispielen in Figuren einer Zeichnung gezeigt und nachfolgend erläutert.

Dabei zeigen:
- FIG. 1: eine erste Ausführungsform eines Ventils mit einem magnetischen Ventilantrieb im Querschnitt schematisch,
- FIG. 2: die Ventilanordnung aus der FIG. 1 in einer dreidimensionalen Ansicht,
- FIG. 3: in einem schematischen Schnitt eine zweite Ausführungsform des erfindungsgemäßen Ventils,
- FIG. 4: eine Ansicht eines elastischen Federelementes,
- FIG. 5: eine dreidimensionale Ansicht der Ventilanordnung aus der FIG. 3,
- FIG. 6: ein Ventil, das mit einer Separiereinrichtung verbunden ist,
- FIG. 7: ein weiteres mit einer Separiereinrichtung verbundenes Ventil,
- FIG. 8: eine Antriebseinheit für ein Funktionselement, das mittels einer in einem Katheter rotierenden Welle antreibbar ist,
- FIG. 9: eine Abwandlung einer Antriebseinheit nach FIG. 8,
- FIG. 10 und FIG. 11: jeweils weitere Gestaltungsformen von Antriebseinrichtungen für in einem Katheter rotierende Wellen sowie
- FIG. 12: eine Abwandlung einer Antriebseinheit nach FIG. 9.

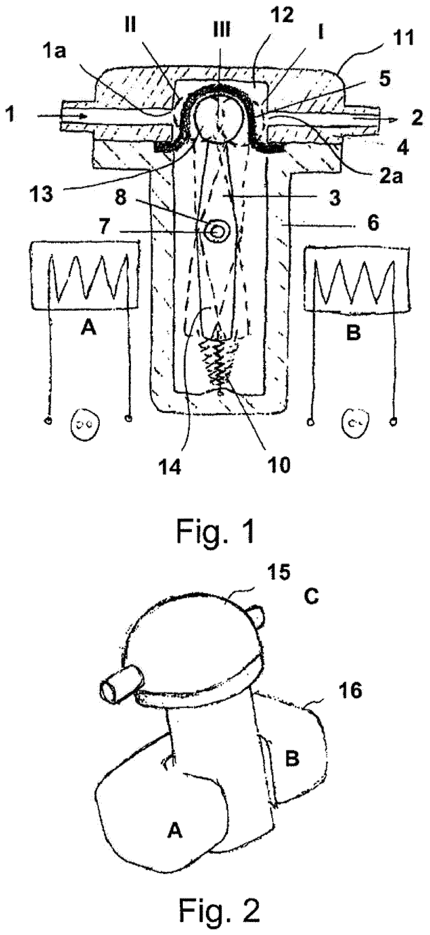

Die FIG. 1 zeigt schematisch einen Ventilkörper 11 mit einem Zuflusskanal 1, einem Abflusskanal 2 sowie einem Antriebshebel 3, der eine Membran 5 auslenkt. Die Membran 5 schließt den Ventilsteuerraum 12, der sich innerhalb des Ventilkörpers 11 befindet, fluiddicht ab und ist durch ein kugelförmiges Ende 13 wahlweise gegen die Zuflussöffnung 1a oder die Abflussöffnung 2a pressbar, um entweder den Zuflusskanal 1 oder den Abflusskanal 2 zu verschließen.

Der Antriebshebel 3 ist um eine Welle 7, die mit Abstandshülsen 8 im Antriebsgehäuse 6 gelagert ist, schwenkbar. Der Antriebshebel 3 ist einmal durchgezogen in der dritten Stellung III dargestellt, in der er einen Verbindungskanal zwischen der Zuflussöffnung 1a und der Abflussöffnung 2a offen lässt, sowie gestrichelt in einer ersten Stellung I, in der das kugelförmige Ende 13 mittels der Membran 5 die Abflussöffnung 2a verschließt, und ebenso gestrichelt in einer zweiten Stellung II, in der der Antriebshebel mittels der Membran 5 die Zuflussöffnung 1a verschließt.

In dem Antriebsgehäuse 6 ist im unteren Teil das elastische Federelement 10 als Schraubenfeder dargestellt, die das der Membran abgewandte Ende des Antriebhebels 3 mit dem Boden des Gehäuses 6 verbindet und somit den Antriebshebel in der dritten Stellung III hält.

Beiderseits des Antriebsgehäuses 6 sind zwei Elektromagneten A, B dargestellt, die, wenn sie mit einem Strom beaufschlagt werden, ein Magnetfeld erzeugen, das auf den unteren Teil 14 des Antriebshebels 3 wirkt und diesen je nach Richtung der magnetischen Kräfte in die erste Stellung I oder in die zweite Stellung II bewegt. Der untere Teil 14 des Antriebshebels 3 ist zu diesem Zweck magnetisch aktiv ausgebildet, entweder als ferromagnetisches, magnetisierbares oder als magnetisiertes Bauteil.

Die FIG. 2 zeigt in einer dreidimensionalen Ansicht ein erstes Gehäuse 15, das den Ventilkörper mit dem Ventilsteuerraum sowie die Membran und zumindest Teile des Antriebshebels 3 und insbesondere auch das Antriebsgehäuse 6 umfasst bzw. beherbergt. Die Magneten A, B sind in einem zweiten Gehäuse 16 angeordnet, das gegenüber dem ersten Gehäuse 15 beweglich, insbesondere auch von diesem separierbar Ist. Es kann vorgesehen sein, dass die beiden Gehäuse 15, 16 verbunden werden, dass beispielsweise das erste Gehäuse 15 in einer Halterung des zweiten Gehäuses 16 einrastbar ist. Jedoch erweist es sich als vorteilhaft, wenn das erste Gehäuse 15 separat entnehmbar ist, so dass die in dem Gehäuse 15 enthaltenen Teile der Ventilvorrichtung gesondert ausgetauscht und insbesondere als Einweg-Ventilteil behandelt werden können.

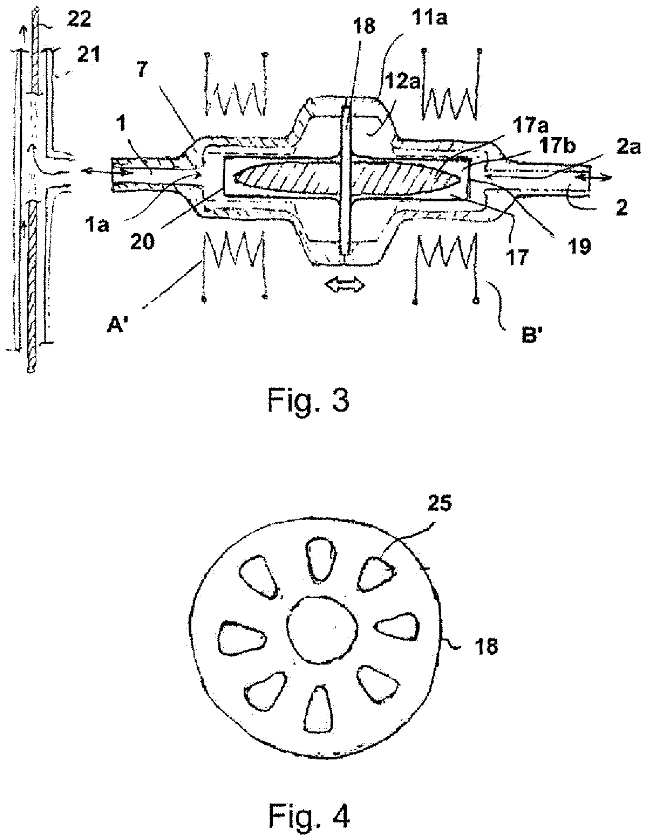

In der FIG. 3 ist eine Ventilanordnung mit einem Ventilkörper 11a gezeigt, der einen Ventilsteuerraum 12a umschließt, in dem ein Verschlusskörper 17 bewegbar gelagert ist. Der Verschlusskörper 17 weist einen magnetisch aktiven Kern 17a und eine den Kern 17a umschließende Umhüllung 17b, insbesondere aus einem Kunststoff, auf und ist mit einer Lagerscheibe 18 verbunden. Die Lagerscheibe ist in einer Ansicht in der FIG. 4 dargestellt. Sie ist an ihrem Umfang am Ventilkörper 11a gelagert und insgesamt elastisch, so dass sie den Verschlusskörper 17 in der dargestellten Mittelstellung hält. Die Lagerscheibe 18 weist mehrere Öffnungen 25 auf, die das Durchströmen des durch das Ventil zu steuernden Fluids erlauben.

Die Enden 19, 20 des Verschlusskörpers 17 sind derart geformt und gestaltet, dass sie die Zuflussöffnung 1a bzw. die Abflussöffnung 2a bei entsprechender Auslenkung des Verschlusskörpers 17 und federnder Verformung der Lagerscheibe 18 verschließen können.

Zu diesem Zweck kann die Umhüllung 17b des Verschlusskörpers 17 beispielsweise aus einem elastischen Stoff, insbesondere einem Elastomer, bestehen. Der magnetische Kern 17a des Verschlusskörpers 17 ist durch die Magneteinrichtungen A', B' mit einer Kraft beaufschlagbar, die den Verschlusskörper 17 entweder in Richtung der Zuflussöffnung 1a oder in Richtung der Abflussöffnung 2a zieht, um das Ventil in die erste oder zweite Verschlussstellung zu bringen.

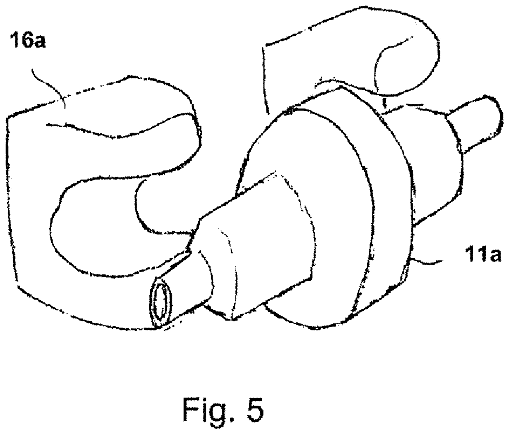

In der FIG. 5 ist in einer dreidimensionalen Ansicht der Ventilkörper 11a als Gehäuse dargestellt, das den Ventilsteuerraum 12a sowie die Zuflusskanal 1 und den Abflusskanal 2 umschließt, sowie das Gehäuse 16a, das die Magneteinrichtungen A' und B' enthält. Das Gehäuse 11a ist, wenn ein Teil der Ventileinrichtung als Einwegventil verwendet werden soll, von dem Gehäuseteil 16a trennbar, so dass die Magneteinrichtungen mehrfach oder weiter verwendet werden können, während der Teil des Ventils, der den Ventilsteuerraum 12a enthält, ausgewechselt werden kann.

Die FIG. 3 zeigt das erfindungsgemäße Ventil im Zusammenhang mit einer ebenfalls erfindungsgemäßen Fluidsteuereinrichtung, die einen Katheter 21, eine den Katheter 21 durchsetzende rotierende Welle 22 sowie eine nicht im Einzelnen dargestellte Spüleinrichtung umfasst, wobei das Ventil mit dem Ventilkörper 11a, dem Ventilsteuerraum 12a und den mechanischen Teilen des Ventilantriebs einen Teil der Spüleinrichtung bildet. Weitere Teile der Spüleinrichtung können beispielsweise eine Spülmittelpumpe und Spülmittelreservoir sein, die im Einzelnen nicht dargestellt sind. Der Zu- oder Abflusskanal 1, 2 kann dann mit dem Katheter 21 verbunden sein, um Spülmittel durch entsprechende Betätigung des Ventils in den Katheter 21 ein- oder aus diesem ausleiten zu können. Es ist in der FIG. 3 auch die verseilte Struktur der Welle 22 zu erkennen, die je nach Geschwindigkeit der Wellenrotation und Abnutzung der Welle zu einer unterschiedlichen Sog- oder Druckwirkung führen kann.

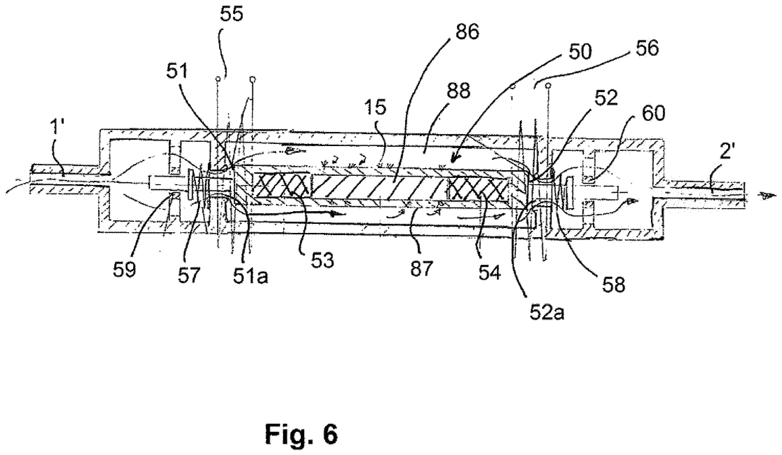

FIG. 6 zeigt ein Magnetventil mit einem Transportkanal, der von einem Fluid zwischen einer Zuflussöffnung 1' und einer Abflussöffnung 2' durchströmt wird. Ein Verschlusskörper 50 ist innerhalb des Transportkanals 88 zwischen einer ersten Verschlussstellung und einer zweiten Verschlussstellung antreibbar, wobei in der ersten Verschlussstellung eine erste Verschlussfläche 51 eine Ventilöffnung 51a verschließt, während in der zweiten Verschlussstellung eine Verschlussfläche 52 eine Ventilöffnung 52 a verschließt.

In den Verschlusskörper 50 sind zwei Ankerkörper 53, 54 integriert, die durch das Magnetfeld von zwei Ventilantriebsspulen 55, 56 antreibbar sind. Axial zwischen den Ankerkörpern 53, 54 ist fluchtend mit diesen der Magnet 86 der Separiereinrichtung angeordnet. Die Ankerkörper sind mit dem Magnetkörper 86 mit einer gemeinsamen Feststoffumhüllung 87 versehen. Beispielhaft sind Partikel, die an der Feststoffumhüllung 87 haften, mit 15 bezeichnet.

Haltefedern 57, 58 halten den Verschlusskörper in Abwesenheit einer Erregung der Ventilantriebsspulen in einer Mittelstellung, in der das Ventil geöffnet ist. Zur Führung des Verschlusskörpers 50 sind zwei Gleitlager 59, 60 an den Enden des Ventilgehäuses vorgesehen.

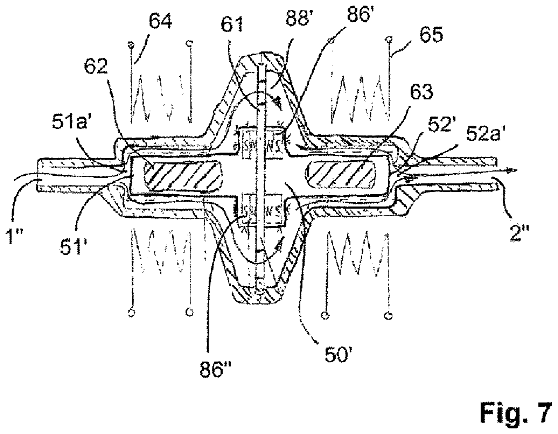

FIG. 7 zeigt ein Ventil mit einer Zuflussöffnung 1", einer Abflussöffnung 2" und einem Verschlusskörper 50'. Der Verschlusskörper 50' ist innerhalb des Transportkanals 88' zwischen einer ersten Verschlussstellung und einer zweiten Verschlussstellung antreibbar, wobei in der ersten Verschlussstellung eine erste Verschlussfläche 51' eine Ventilöffnung 51a' verschließt, während in der zweiten Verschlussstellung eine Verschlussfläche 52' eine Ventilöffnung 52a' verschließt. Der Verschlusskörper 50' ist mittels einer elastischen, durchlässigen Scheibe 61 in dem Gehäuse des Ventils gelagert und in einer geöffneten Mittelstellung gehalten. Die Scheibe 61 trägt Separiermagnete 86', 86", die in dem Verschlusskörper 50' mit Ventilantriebsankern 62, 63 verbunden und gemeinsam mit diesen von einer Schutzschicht umhüllt sind.

Die Ventilantriebsanker 62, 63 sind im Feld der Spulen 64, 65 antreibbar. Partikel können sich im Transportkanal an den Separiermagneten auf der Schutzschicht anlagern und werden dort festgehalten.

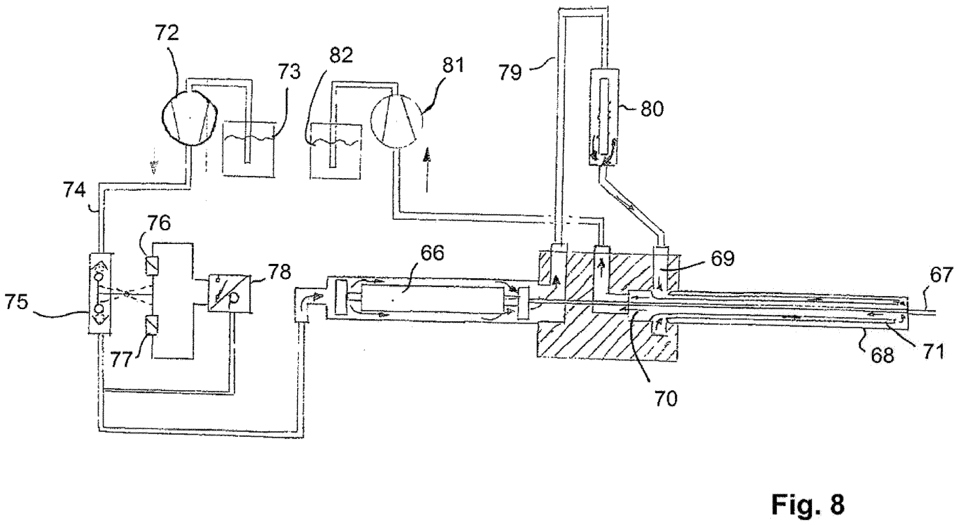

Die in den Ansprüchen genannte Kathetervorrichtung 100 wird nun in den FIGN. 8-12 in mehreren Alternativen gezeigt.

Die FIG. 8 zeigt eine Kathetervorrichtung 100, enthaltend eine Antriebseinrichtung mit einem rotierend antreibbaren Antriebsanker 66, der eine rotierende Welle 67 in einem Katheter 68 antreibt. Innerhalb des Katheters 68 sind Lumina, ausgeführt als ein Zuströmkanal 69 radial außen und ein Rückstromkanal 70 radial innen, konzentrisch zueinander und zur Außenhülle des Katheters angeordnet. Der Zuströmkanal 69 und der Rückströmkanal 70 sind durch eine schlauchartige Trennwand 71 voneinander getrennt.

Durch eine volumengesteuerte Schlauchpumpe 72 wird eine Spülflüssigkeit von einem Reservoir 73 durch ein als eine Kanüle ausgeführtes Lumen 74 und ein Ventil 75 gepumpt. Zwei Magnete 76 und 77 dienen dem Antrieb des Ventils und werden mittels eines Druckschalters 78 angesteuert mit dem Ziel, einen gleichbleibenden Druck im Zuströmkanal 69 aufrecht zu erhalten. Hierzu wird das Fluid durch das Ventil 75 und durch das Gehäuse des Antriebsankers 66, das als Transportkanal ausgeführte Lumen 79 und durch die Separiereinrichtung 80 geleitet, wo aktiv Partikel aus dem Fluid ausgefiltert werden. Die Separiereinrichtung 80 kann so aufgebaut sein wie die in der FIG. 6 gezeigte Separiereinrichtung. Von dort strömt das Fluid in den Katheter 68 durch den Zuströmkanal 69 radial außen und den Rückstromkanal 70 radial innen sowie von dort zu einer Schlauchpumpe 81, die das Fluid ansaugt und in das Reservoir 82 leitet. Die Schlauchpumpe 81 kann jedoch auch der Rückspülung dienen und zu diesem Zweck derart betrieben werden, dass sie das Fluid zum Rücklaufkanal 70 und von dort über den Zuströmkanal 69, durch die Separiereinrichtung zurück zum Ventil 75 in das Reservoir 73 fördert, um beispielsweise die eingefangenen Partikel von der Separiereinrichtung zu entfernen.

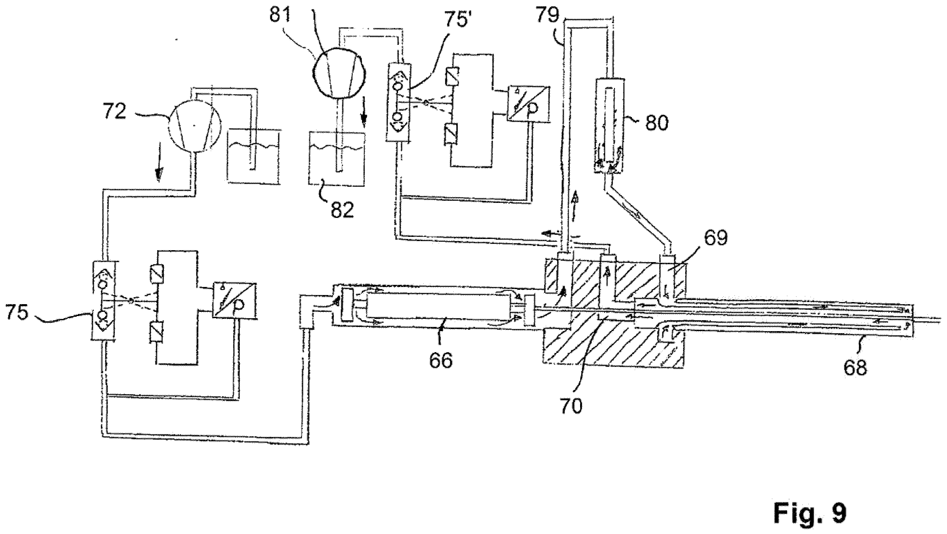

Die FIG. 9 zeigt einen Aufbau ähnlich der aus FIG. 8, wobei zusätzlich zu dem Ventil 75 vor dem Antriebsanker 66 und hinter der Schlauchpumpe 72 ein zweites Ventil 75' zwischen dem Rückströmkanal 70 und der Rückströmpumpe 80 angeordnet ist. Während FIG.8 bei Spülsystemen Anwendung findet, in denen durch Anlagenkomponenten kein ungewollter Unterdruck im Rücklauf erzeugt wird, ist es möglich, FIG.9 auch bei Spülsystemen einzusetzen, in denen im Rücklauf ein ungewollter Unterdruck entsteht (z. B. durch Wicklungsrichtung einer biegsamen Welle). Dieser Unterdruck wird vom Sensor erkannt, der dann durch Schließen des Ventils 75' nach unten dafür sorgt, dass kein Medium aus Behälter 82 über die Pumpe 81 in den Spülkreislauf kommt. Die Separiereinrichtung ist somit zwischen zwei Ventilen und auch zwischen zwei Fluidfördereinrichtungen angeordnet, von denen wenigstens eines, insbesondere beide, bezüglich der Förderrichtung des Fluides umschaltbar sind, um die Strömungsrichtung umzukehren.

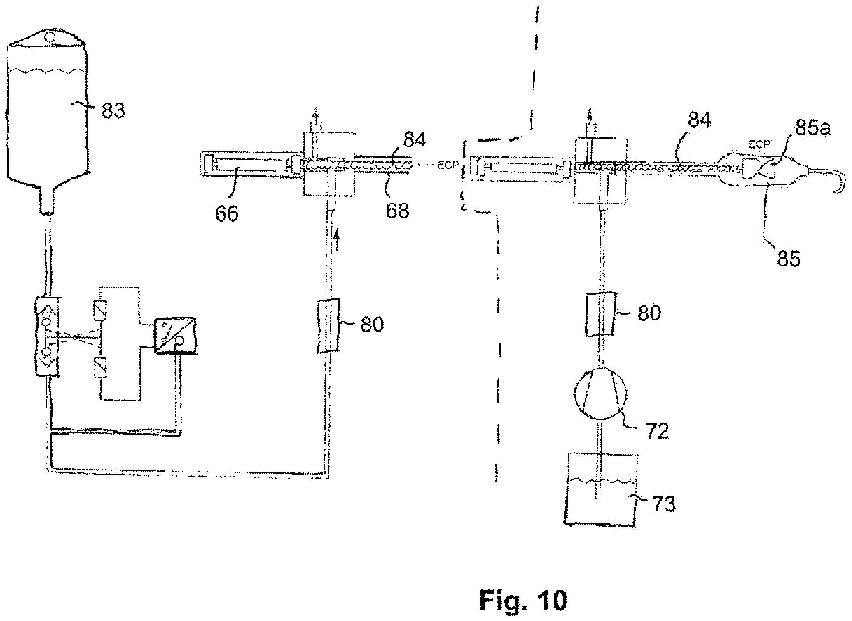

Bei dem Aufbau gemäß FIG. 10 ist gegenüber dem Aufbau in FIG. 8 lediglich eine Schlauchpumpe 72 durch ein Reservoir 83 ersetzt, das eine Schwerkraftspülung erlaubt, indem das Fluid durch die Schwerkraftdurch das Ventil 75 und weiter zum Katheter 68 fließt. Die rotierende Welle 84 innerhalb des Katheters 68 weist durch ihren verseilten Aufbau auf der Basis von verseilten Litzen eine gewendelte Außenstruktur auf, die ihr selbst bei Rotation eine Pumpwirkung in Richtung vom Antriebsanker 66 weg verleiht. Auf der rechten Seite der Figur 10, rechts von der gestrichelten Linie 85, ist für den Zufluss von Fluid zum Katheter 68 eine andere Variante mit einer volumengesteuerten Schlauchpumpe 72 und einem Reservoir 73 dargestellt. Die Schlauchpumpe fördert dort das Fluid zum Inneren des Katheters, der beispielsweise in den Körper eines Patienten hineinführt und dort an einer Herzpumpe 85 mit einem Rotor 85a endet. Die Herzpumpe kann beispielsweise radial komprimierbar bzw. insgesamt besonders anfällig für Partikel sein, die hineingeraten. Von dort strömt das Fluid dann zurück. Eine Separiereinrichtung 80 kann jeweils in Strömungsrichtung vor dem Katheter 68 zwischen diesem und der Fördereinrichtung 73, 83, insbesondere jedenfalls vor der Herzpumpe 85 vorgesehen sein.

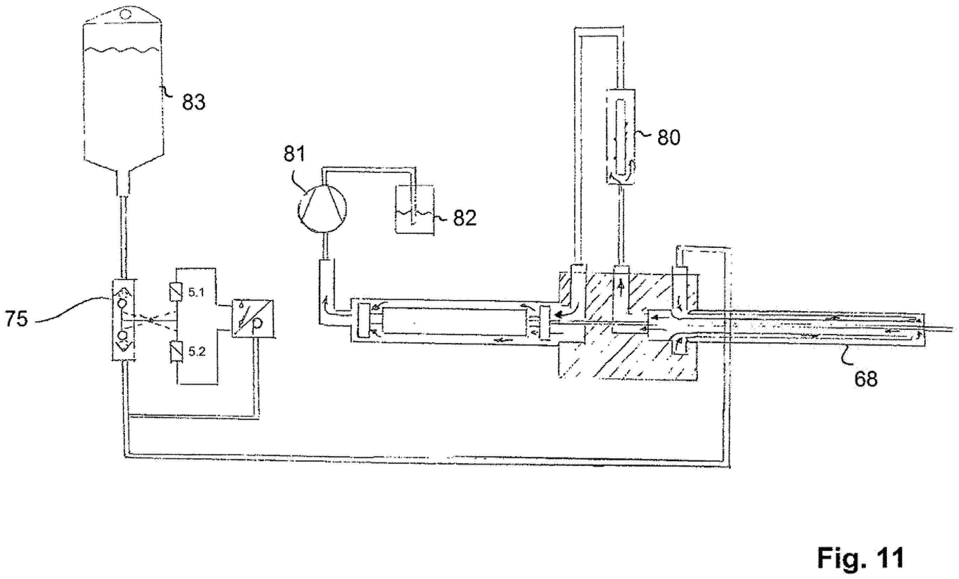

Die FIG. 11 zeigt eine Konstellation ähnlich der aus Figur 9, wobei anstelle der Schlauchpumpe 72 eine Schwerkraftförderung 83 vorgesehen ist, wobei das Fluid im Normalbetrieb von dort über das Ventil in den Katheter 68 und dort zunächst durch den Zuströmkanal 69 radial außen in den Rückstromkanal 70 radial innen sowie von dort zu einer Schlauchpumpe 81, die das Fluid ansaugt und in das Reservoir 82 leitet. Zwischen dem Rückstromkanal 70 und der Schlauchpumpe 81 passiert das Fluid zunächst die Separiereinrichtung 80, die zwischen dem Rückstromkanal und dem Gehäuse des Antriebsankers 66 angeordnet ist. Danach strömt das Fluid am Antriebsanker 66 vorbei zur Schlauchpumpe 81. Die Lagerung des Antriebsankers kann relativ unempfindlich sein, so dass die Durchströmrichtung des Fluids dort von untergeordneter Bedeutung ist. Wichtig ist besonders, dass das Gehäuse des Antriebsankers mit dem Fluid versorgt ist, um eine gute Schmierung zu gewährleisten. Die gewählte Anordnung stellt darüber hinaus sicher, dass magnetische Abriebpartikel der rotierenden Welle 84 in diesem Fall nicht die Lager des Antriebsankers beschädigen können.

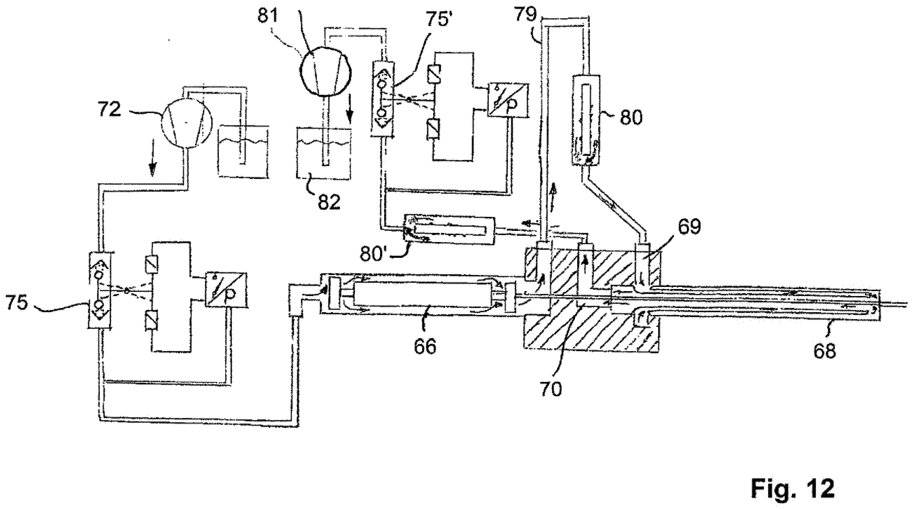

Die FIG. 12 zeigt einen Aufbau ähnlich der FIG. 9, wobei eine weitere Separiereinrichtung 80'dafür sorgt, dass die Funktion der Dichtflächen des Ventils 75' nicht durch anhaftende Partikel beeinträchtigt wird.

Die Erfindung ist durch die Ansprüche definiert. Die nachfolgenden Aspekte sind für das Verständnis der Erfindung hilfreich und bilden Teil der Offenbarung:
1. Ventil zur Steuerung eines Fluidflusses insbesondere durch einen Katheter, mit einem Ventilsteuerraum (12, 12a), in dem ein Zuflusskanal (1) mit einer Zuflussöffnung (1a) und ein Abflusskanal (2) mit einer Abflussöffnung (2a) münden, und mit einem in dem Ventilsteuerraum (12, 12a) gesteuert bewegbaren Verschlusselement (5, 13, 17), das in wenigstens einer ersten Stellung (I) die Abflussöffnung (2a), in wenigstens einer zweiten Stellung (II) die Zuflussöffnung (1a) verschließt und das in wenigstens einer dritten Stellung (III) einen Verbindungskanal zwischen der Zuflussöffnung (1a) und der AbflussÖffnung (2a) offenhält, wobei ein Ventilantrieb (A, B, A', B', 3, 14, 18) vorgesehen ist, der das Verschlusselement (5, 13, 17) wahlweise wenigstens in die erste, zweite oder dritte Stellung bewegt.
2. Ventil nach Aspekt 1,
   *dadurch gekennzeichnet, dass*
   die dritte Stellung (III) des Verschlusselementes (5, 13, 17) zwischen der ersten und der zweiten Stellung liegt.
3. Ventil nach Aspekt 1 oder 2,
   *dadurch gekennzeichnet, dass*
   der Ventilsteuerraum (12, 12a) von einem Ventilantrieb des Verschlusselements (5,13, 17) separierbar ist.
4. Ventil nach Aspekt 1 oder einem der folgenden,
   *dadurch gekennzeichnet, dass*
   der Ventilsteuerraum (12, 12a) mit Ausnahme der Zuflussöffnung (1a) und der Abflussöffnung (2a) allseits fluiddicht abgeschlossen ist.
5. Ventil nach Aspekt 1 oder einem der folgenden,
   *dadurch gekennzeichnet, dass*
   das Verschlusselement (5, 13, 17) eine bewegliche Membran (5) aufweist, die den Ventilsteuerraum (12) fluiddicht abschließt und derart auslenkbar ist, dass so wahlweise die Zuflussöffnung (1a) oder die Abflussöffnung (2a) durch Teile der Membran (5) verschließbar sind.
6. Ventil nach Aspekt 5,
   *dadurch gekennzeichnet, dass*
   ein Antriebshebel (3, 14) des Ventilantriebs (3, 14, 18, A, A', B, B') die Membran (5) wenigstens in der ersten und zweiten Stellung auslenkt.
7. Ventil nach Aspekt 1 oder einem der folgenden,
   *dadurch gekennzeichnet, dass*
   das Verschlusselement (5, 13, 17) durch einen magnetisch wirkenden Ventilantrieb (A, A', B, B') antreibbar ist.
8. Ventil nach Aspekt 7,
   *dadurch gekennzeichnet, dass*
   das in dem Ventilsteuerraum (12, 12a) angeordnete Verschlusselement (S, 13, 17) magnetisch aktiv ist und mit einem Magnetfeld des Ventilantriebs (A, A', B, B') wechselwirkt.
9. Ventil nach Aspekt 6 und 7,
   *dadurch gekennzeichnet, dass*
   der Antriebshebel (3, 14) magnetisch antreibbar ist.
10. Ventil nach Aspekt 7,
   *dadurch gekennzeichnet, dass*
   sowohl der Ventilsteuerraum (12, 12a) als auch die mit dem Verschlusselement (5, 13, 17) mechanisch verbundenen Teile (3, 14, 18) des Ventilantriebs mit Ausnahme der Zufluss- und Abflussöffnung (1a, 2a) fluiddicht abgeschlossen und von einer Magnetfelderzeugungseinrichtung (A, A', B, B') des Ventilantriebs separierbar sind.
11. Ventil nach Aspekt 1 oder einem der folgenden,
   *dadurch gekennzeichnet, dass*
   das Verschlusselement durch ein elastisches Federelement (10, 18) vorzugsweise in die dritte Stellung (III) bewegt wird.
12. Ventil nach Aspekt 1 oder einem der folgenden, dadurch gekennzeichnet, dass unmittelbar am Ventilsteuerraum (12, 12a), insbesondere im Inneren des Verschlusselements (5, 13, 17), ein Magnet (13", 13‴, 13ʺʺ) als Teil einer Separiereinrichtung vorgesehen ist.
13. Ventil nach Aspekt 12, dadurch gekennzeichnet, dass der/die Magnet(e) (13", 13‴, 13ʺʺ) von Antriebsankern (53, 54, 62, 63) des einen Ventilantriebs separat, insbesondere beabstandet, vorgesehen sind.
14. Katheter, enthaltend ein Ventil nach mindestens einem der vorhergehenden Aspekte, dadurch gekennzeichnet, dass der Ventilsteuerraum von einem Ventilantrieb des Verschlusselements separierbar ist.
15. Schutzeinrichtung für ein Ventil (75, 75'), das mit einem strömenden Fluid in Verbindung steht, dadurch gekennzeichnet, dass entlang eines Strömungskanals (79, 88) für das Fluid, insbesondere eines Katheters, von dem Ventil beabstandet und insbesondere von diesem separiert eine Separiereinrichtung (80) zur Rückhaltung von im Fluid befindlichen Partikeln mit wenigstens einem Magnetelement (86, 86', 86") vorgesehen ist.

Des Weiteren sind auch die weiteren nachfolgenden Aspekte, die sich auf eine Kathetervorrichtung beziehen, Teil der Offenbarung und ebenfalls für das Verständnis der Erfindung hilfreich:
1. Kathetervorrichtung (100), enthaltend
   einen Katheter (68) zum Einführen in ein Lebewesen sowie mindestens ein Lumen (69, 70, 74, 79) zum Führen eines Fluidflusses innerhalb eines Teils der Kathetervorrichtung sowie
   enthaltend ein Ventil zur Steuerung eines Fluidflusses insbesondere durch einen Katheter, mit einem Ventilsteuerraum (12, 12a), in dem ein Zuflusskanal (1) mit einer Zuflussöffnung (1a) und ein Abflusskanal (2) mit einer Abflussöffnung (2a) münden, und mit einem in dem Ventilsteuerraum (12, 12a) gesteuert bewegbaren Verschlusselement (5, 13, 17), das in wenigstens einer ersten Stellung (I) die Abflussöffnung (2a), in wenigstens einer zweiten Stellung (II) die Zuflussöffnung (1a) verschließt und das in wenigstens einer dritten Stellung (III) einen Verbindungskanal zwischen der Zuflussöffnung (1a) und der Abflussöffnung (2a) offenhält, wobei ein Ventilantrieb (A, B, A', B', 3, 14, 18) vorgesehen ist, der das Verschlusselement (5, 13, 17) wahlweise wenigstens in die erste, zweite oder dritte Stellung bewegt,
   wobei das mindestens eine Lumen (69, 70, 74, 79) mit dem Zuflusskanal oder dem Abflusskanal fluidleitend verbunden ist.
2. Kathetervorrichtung nach Aspekt 1,
   dadurch gekennzeichnet, dass
   der Katheter (68) eine drehbare Welle aufweist.
3. Kathetervorrichtung nach Aspekt 2,
   dadurch gekennzeichnet, dass
   die drehbare Welle (67) flexibel ist, insbesondere derart flexibel, dass sie an die Krümmung eines menschlichen Aortenbogens anpassbar ist.
4. Kathetervorrichtung nach Aspekt 2 oder 3,
   dadurch gekennzeichnet, dass
   eine außerhalb des Lebewesens platzierbare Antriebseinrichtung (66, 67) zum Drehen der Welle einsetzbar ist.
5. Kathetervorrichtung nach einem der vorhergehenden Aspekte,
   dadurch gekennzeichnet, dass
   der Katheter (68) als Katheterpumpe, insbesondere als expandierbare Katheterpumpe ausgeführt ist.
6. Kathetervorrichtung nach einem der vorhergehenden Aspekte,
   dadurch gekennzeichnet, dass
   der Katheter (68) mehr als ein Lumen (69, 70, 74, 79) aufweist, wobei mindestens ein Lumen (69) für die Fluidführung in distaler Richtung und mindestens ein Lumen (70) für die Fluidführung in proximaler Richtung ausgeführt ist.
7. Kathetervorrichtung nach Aspekt 6,
   dadurch gekennzeichnet, dass
   zwischen 10 und 90 Prozent des in distaler Richtung in einem ersten Lumen geführten Fluidstroms in einem anderen Lumen (70) in proximaler Richtung rückführbar sind.
8. Kathetervorrichtung nach Aspekt 4,
   dadurch gekennzeichnet, dass
   ein Lumen zumindest teilweise durch die Antriebseinrichtung (66, 67) geführt ist.
9. Kathetervorrichtung nach einem der vorhergehenden Aspekte,
   dadurch gekennzeichnet, dass
   ein, zwei, drei oder mehr Ventile (75, 75') vorgesehen sind.
10. Kathetervorrichtung nach einem der vorhergehenden Aspekte,
   dadurch gekennzeichnet, dass
   ein erstes Ventil vorgesehen ist, wobei der Abfluss des ersten Ventils (75) mit dem Zufluss zur Kathetervorrichtung verbunden ist und ein zweites Ventil (75') vorhanden ist, wobei der Zufluss des zweiten Ventils (75') mit einem in proximaler Richtung fluidführenden Lumen (69) verbunden ist.
11. Kathetervorrichtung nach einem der vorhergehenden Aspekte,
   dadurch gekennzeichnet, dass
   die dritte Stellung (III) des Verschlusselementes (5, 13, 17) zwischen der ersten und der zweiten Stellung liegt.
12. Kathetervorrichtung nach einem der vorhergehenden Aspekte,
   dadurch gekennzeichnet, dass
   der Ventilsteuerraum (12, 12a) von einem Ventilantrieb des Verschlusselements (5, 13, 17) separierbar ist.
13. Kathetervorrichtung nach einem der vorhergehenden Aspekte,
   dadurch gekennzeichnet, dass
   der Ventilsteuerraum (12, 12a) mit Ausnahme der Zuflussöffnung (1a) und der Abflussöffnung (2a) allseits fluiddicht abgeschlossen ist.
14. Kathetervorrichtung nach einem der vorhergehenden Aspekte,
   dadurch gekennzeichnet, dass
   das Verschlusselement (5, 13, 17) eine bewegliche Membran (5) aufweist, die den Ventilsteuerraum (12) fluiddicht abschließt und derart auslenkbar ist, dass so wahlweise die Zuflussöffnung (1a) oder die Abflussöffnung (2a) durch Teile der Membran (5) verschließbar sind.
15. Kathetervorrichtung nach Aspekt 14,
   dadurch gekennzeichnet, dass
   ein Antriebshebel (3, 14) des Ventilantriebs (3, 14, 18, A, A', B, B') die Membran (5) wenigstens in der ersten und zweiten Stellung auslenkt.
16. Kathetervorrichtung nach einem der vorhergehenden Aspekte,
   dadurch gekennzeichnet, dass
   das Verschlusselement (5, 13, 17) durch einen magnetisch wirkenden Ventilantrieb (A, A', B, B') antreibbar ist.
17. Kathetervorrichtung nach Aspekt 16,
   dadurch gekennzeichnet, dass
   das in dem Ventilsteuerraum (12, 12a) angeordnete Verschlusselement (5, 13, 17) magnetisch aktiv ist und mit einem Magnetfeld des Ventilantriebs (A, A', B, B') wechselwirkt.
18. Kathetervorrichtung nach Aspekt 15 und 16,
   dadurch gekennzeichnet, dass
   der Antriebshebel (3, 14) magnetisch antreibbar ist.
19. Kathetervorrichtung nach Aspekt 16,
   dadurch gekennzeichnet, dass
   sowohl der Ventilsteuerraum (12, 12a) als auch die mit dem Verschlusselement (5, 13, 17) mechanisch verbundenen Teile (3, 14, 18} des Ventilantriebs mit Ausnahme der Zufluss- und Abflussöffnung (1a, 2a) fluiddicht abgeschlossen und von einer Magnetfelderzeugungseinrichtung (A, A', B, B') des Ventilantriebs separierbar sind.
20. Kathetervorrichtung nach einem der vorhergehenden Aspekte,
   dadurch gekennzeichnet, dass
   das Verschlusselement durch ein elastisches Federelement (10, 18) vorzugsweise in die dritte Stellung (III) bewegt wird.
21. Kathetervorrichtung nach einem der vorhergehenden Aspekte, dadurch gekennzeichnet, dass unmittelbar am Ventilsteuerraum (12, 12a), insbesondere im Inneren des Verschlusselements (5, 13, 17), ein Magnet (13", 13‴, 13"") als Teil einer Separiereinrichtung vorgesehen ist.
22. Kathetervorrichtung nach Aspekt 21, dadurch gekennzeichnet, dass der/die Magnet(e) (13", 13‴, 13"") von Antriebsankern (53, 54, 62, 63) des einen Ventilantriebs separat, insbesondere beabstandet, vorgesehen sind.
23. Katheter, enthaltend ein Ventil nach mindestens einem der vorhergehenden Aspekte, dadurch gekennzeichnet, dass der Ventilsteuerraum von einem Ventilantrieb des Verschlusselements separierbar ist.
24. Schutzeinrichtung für ein Ventil (75, 75'), das mit einem strömenden Fluid in Verbindung steht, dadurch gekennzeichnet, dass entlang eines Strömungskanals (79, 88) für das Fluid, insbesondere eines Katheters, von dem Ventil beabstandet und insbesondere von diesem separiert eine Separiereinrichtung (80) zur Rückhaltung von im Fluid befindlichen Partikeln mit wenigstens einem Magnetelement (86, 86',86") vorgesehen ist.

## Patentansprüche

1. Kathetereinrichtung mit
- einem Ventil (75, 75') oder zwei Ventilen, das/die mit einem strömenden Fluid in Verbindung steht/stehen,
- einem Strömungskanal (79, 88), der als Katheter (21) in Form einer Katheterpumpe ausgeführt ist und eine drehbare Welle (22) aufweist, und
- einer Schutzvorrichtung für das eine Ventil (75, 75') oder die zwei Ventile, wobei entlang des Strömungskanals (79, 88) für das Fluid, von dem Ventil/den Ventilen (75, 75') beabstandet und insbesondere von diesem/diesen separiert, eine Separiereinrichtung (80) zur Rückhaltung von im Fluid befindlichen Partikeln mit wenigstens einem Magnetelement (86, 86',86") vorgesehen ist.

2. Kathetervorrichtung nach Anspruch 1, **dadurch gekennzeichnet, dass** der Strömungskanal (79, 88) als Katheter (21) in Form einer expandierbaren Katheterpumpe ausgeführt ist.

3. Kathetervorrichtung nach Anspruch 1 oder 2, wobei der Katheter (21) die drehbare Welle (22) aufweist und wobei die drehbare Welle (22) derart flexibel ist, dass sie an eine Krümmung des menschlichen Aortenbogens anpassbar ist.

4. Kathetervorrichtung nach einem der vorangehenden Ansprüche, enthaltend wenigstens ein Ventil (75, 75') zur Steuerung eines Fluidflusses, insbesondere durch einen Katheter, mit einem Ventilsteuerraum (12, 12a), in den ein Zuflusskanal (1) mit
einer Zuflussöffnung (1a) und ein Abflusskanal (2) mit einer Abflussöffnung (2a) münden, und mit einem in dem Ventilsteuerraum (12, 12a) gesteuert bewegbaren Verschlusselement (5, 13, 17), das in wenigstens einer ersten Stellung (I) die Abflussöffnung (2a), in wenigstens einer zweiten Stellung (II) die Zuflussöffnung (1a) verschließt und das in wenigstens einer dritten Stellung (III) einen Verbindungskanal zwischen der Zuflussöffnung (1a) und der Abflussöffnung (2a) offenhält, wobei ein Ventilantrieb (A, B, A', B', 3, 14, 18) vorgesehen ist, der das Verschlusselement (5, 13, 17) wahlweise wenigstens in die erste, zweite oder dritte Stellung bewegt.

5. Kathetervorrichtung nach Anspruch 4, **dadurch gekennzeichnet, dass** die dritte Stellung (III) des Verschlusselementes (5, 13, 17) zwischen der ersten und der zweiten Stellung liegt.

6. Kathetervorrichtung nach Anspruch 4 oder 5, **dadurch gekennzeichnet, dass** der Ventilsteuerraum (12, 12a) von einem Ventilantrieb des Verschlusselements (5, 13, 17) separierbar ist.

7. Kathetervorrichtung nach einem der Ansprüche 4, 5 oder 6, **dadurch gekennzeichnet, dass** der Ventilsteuerraum (12, 12a) mit Ausnahme der Zuflussöffnung (1a) und der Abflussöffnung (2a) allseits fluiddicht abgeschlossen ist.

8. Kathetervorrichtung nach einem der Ansprüche 4, 5, 6 oder 7, **dadurch gekennzeichnet, dass** das Verschlusselement (5, 13, 17) eine bewegliche Membran (5) aufweist, die den Ventilsteuerraum (12) fluiddicht abschließt und derart auslenkbar ist, dass so wahlweise die Zuflussöffnung (1a) oder die Abflussöffnung (2a) durch Teile der Membran (5) verschließbar sind.

9. Kathetervorrichtung nach einem der Ansprüche 4 bis 8, **dadurch gekennzeichnet, dass** ein Antriebshebel (3, 14) des Ventilantriebs (3, 14, 18, A, A', B, B') die Membran (5) wenigstens in der ersten und zweiten Stellung auslenkt.

10. Kathetervorrichtung nach einem der Ansprüche 4 bis 9, **dadurch gekennzeichnet, dass** das Verschlusselement (5, 13, 17) durch einen magnetisch wirkenden Ventilantrieb (A, A', B, B') antreibbar ist.

11. Kathetervorrichtung nach Anspruch 9 oder 10, **dadurch gekennzeichnet, dass** das in dem
Ventilsteuerraum (12, 12a) angeordnete Verschlusselement (5, 13, 17) magnetisch aktiv ist und mit einem Magnetfeld des Ventilantriebs (A, A', B, B') wechselwirkt.

12. Kathetervorrichtung nach Anspruch 8 oder 9, **dadurch gekennzeichnet, dass** der Antriebshebel (3, 14) magnetisch antreibbar ist und/oder dass der Ventilsteuerraum von einem Ventilantrieb des Verschlusselements separierbar ist.

13. Kathetervorrichtung nach Anspruch 11 oder 12, **dadurch gekennzeichnet, dass** sowohl der Ventilsteuerraum (12, 12a) als auch die mit dem Verschlusselement (5, 13, 17) mechanisch verbundenen Teile (3, 14, 18) des Ventilantriebs mit Ausnahme der Zufluss- und Abflussöffnung (1a, 2a) fluiddicht abgeschlossen und von einer Magnetfelderzeugungseinrichtung (A, A', B, B') des Ventilantriebs separierbar sind und/oder dass das Verschlusselement durch ein elastisches Federelement (10, 18) vorzugsweise in die dritte Stellung (III) bewegt wird.

14. Kathetervorrichtung nach einem der Ansprüche 4 bis 13, **dadurch gekennzeichnet, dass** unmittelbar am Ventilsteuerraum (12, 12a), insbesondere im Inneren des Verschlusselements (5, 13, 17), ein Magnet (13", 13‴, 13ʺʺ) als Teil einer Separiereinrichtung vorgesehen ist, wobei der/die Magnet(e) (13", 13‴, 13ʺʺ) insbesondere von Antriebsankern (53, 54, 62, 63) des einen Ventilantriebs separat, insbesondere beabstandet, vorgesehen sind.

## Claims

1. A catheter device including
- one valve (75, 75') or two valves that is/are in communication with a flowing fluid,
- a flow channel (79, 88), which is designed as a catheter (21) in the form of a catheter pump and has a rotatable shaft (22), and
- a protective device for the one valve (75, 75') or the two valves, wherein a separating device (80) for retaining particles present in the fluid, with at least one magnetic element (86, 86', 86"), is provided along the flow channel (79, 88) for the fluid, at a distance from the valve(s) (75, 75') and in particular separated therefrom.

2. The catheter device according to claim 1, **characterized in that** the flow channel (79, 88) is designed as a catheter (21) in the form of an expandable catheter pump.

3. The catheter device according to claim 1 or 2, wherein the catheter (21) comprises the rotatable shaft (22) and wherein the rotatable shaft (22) is flexible such that it is adaptable to a curvature of the human aortic arch.

4. The catheter device according to one of the preceding claims, comprising at least one valve (75, 75') for controlling a fluid flow, in particular through a catheter, with a valve control chamber (12, 12a) into which an inflow channel (1) with an inflow opening (1a) and an outflow channel (2) with an outflow opening (2a) run out, and with a closure element (5, 13, 17) which can be moved in a controlled manner in the valve control chamber (12, 12a), which in at least one first position (I) closes the outflow opening (2a), in at least one second position (II) closes the inflow opening (1a) and in at least one third position (III) keeps open a connecting channel between the inflow opening (1a) and the outflow opening (2a), wherein a valve drive (A, B, A', B', 3, 14, 18) is provided which selectively moves the closure element (5, 13, 17) at least into the first, second or third position.

5. The catheter device according to claim 4, **characterized in that** the third position (III) of the closure element (5, 13, 17) lies between the first and the second position.

6. The catheter device according to claim 4 or 5, **characterized in that** the valve control chamber (12, 12a) can be separated from a valve drive of the closure element (5, 13, 17).

7. The catheter device according to one of claims 4, 5 or 6, **characterized in that** the valve control chamber (12, 12a) is sealed fluid-tight on all sides with the exception of the inflow opening (1a) and the outflow opening (2a).

8. The catheter device according to one of claims 4, 5, 6 or 7, **characterized in that** the closure element (5, 13, 17) has a movable diaphragm (5) which closes the valve control chamber (12) in a fluid-tight manner and can be deflected in such a way that the inflow opening (1a) or the outflow opening (2a) can be selectively closed by parts of the diaphragm (5).

9. The catheter device according to one of claims 4 to 8, **characterized in that** a drive lever (3, 14) of the valve drive (3, 14, 18, A, A', B, B') deflects the diaphragm (5) at least in the first and second position.

10. The catheter device according to one of claims 4 to 9, **characterized in that** the closure element (5, 13, 17) can be driven by a magnetically acting valve drive (A, A', B, B').

11. The catheter device according to claim 9 or 10, **characterized in that** the closure element (5, 13, 17) arranged in the valve control chamber (12, 12a) is magnetically active and interacts with a magnetic field of the valve drive (A, A', B, B').

12. The catheter device according to claim 8 or 9, **characterized in that** the drive lever (3, 14) can be driven magnetically and/or **in that** the valve control chamber can be separated from a valve drive of the closure element.

13. The catheter device according to claim 11 or 12, **characterized in that** both the valve control chamber (12, 12a) and the parts (3, 14, 18) of the valve drive mechanically connected to the closure element (5, 13, 17), with the exception of the inflow and outflow opening (1a, 2a) are sealed in a fluid-tight manner and can be separated from a magnetic field generating device (A, A', B, B') of the valve drive and/or that the closure element is preferably moved into the third position (III) by an elastic spring element (10, 18).

14. The catheter device according to one of claims 4 to 13, **characterized in that** a magnet (13", 13‴, 13"") is provided directly on the valve control chamber (12, 12a), in particular in the interior of the closure element (5, 13, 17), as part of a separating device, the magnet(s) (13", 13‴, 13"") in particular being provided separately, in particular at a distance, from drive anchors (53, 54, 62, 63) of the one valve drive.

## Revendications

1. Dispositif de cathéter, comprenant
- une valve (75, 75') ou deux valves qui est/sont reliée(s) à un fluide en écoulement,
- un canal d'écoulement (79, 88) qui est conçu en tant que cathéter (21) sous la forme d'une pompe de cathéter et présente un arbre rotatif (22), et
- un dispositif de protection pour l'une valve (75, 75') ou pour les deux valves, dans lequel un dispositif de séparation (80) pour retenir des particules se trouvant dans le fluide avec au moins un élément magnétique (86, 86', 86") est prévu le long du canal d'écoulement (79, 88) du fluide, à distance de la valve/des valves (75, 75'), et en particulier séparé de celle(s)-ci.

2. Dispositif de cathéter selon la revendication 1, **caractérisé en ce que** le canal d'écoulement (79, 88) est réalisé en tant que cathéter (21) sous la forme d'une pompe de cathéter extensible.

3. Dispositif de cathéter selon la revendication 1 ou 2, dans lequel le cathéter (21) présente la tige rotative (22), et dans lequel la tige rotative (22) est flexible de telle sorte qu'elle peut s'adapter à une courbure de l'arc aortique humain.

4. Dispositif de cathéter selon l'une quelconque des revendications précédentes, contenant au moins une valve (75, 75') pour commander un écoulement de fluide, notamment à travers un cathéter, avec une chambre de commande de valve (12, 12a), dans laquelle débouche un canal d'entrée (1) avec une ouverture d'entrée (1a) et un canal d'évacuation (2) avec une ouverture d'évacuation (2a), et avec un élément de fermeture mobile commandé (5, 13, 17) dans la chambre de commande de valve (12, 12a), qui ferme l'ouverture d'évacuation (2a) dans au moins une première position (I), ferme l'ouverture d'entrée (1a) dans au moins une deuxième position (II) et maintient un canal de liaison entre l'ouverture d'entrée (1a) et l'ouverture de sortie (2a) ouvert dans au moins une troisième position (III), dans lequel un entraînement de valve (A, B, A', B', 3, 14, 18) est prévu, qui déplace sélectivement l'élément de fermeture (5, 13, 17) dans au moins la première, la deuxième ou la troisième position.

5. Dispositif de cathéter selon la revendication 4, **caractérisé en ce que** la troisième position (III) de l'élément de fermeture (5, 13, 17) se situe entre la première et la deuxième position.

6. Dispositif de cathéter selon la revendication 4 ou 5, **caractérisé en ce que** la chambre de commande de valve (12, 12a) peut être séparée d'un entraînement de valve de l'élément de fermeture (5, 13, 17).

7. Dispositif de cathéter selon l'une des revendications 4, 5 ou 6, **caractérisé en ce que** la chambre de commande de valve (12, 12a) est fermée de manière étanche aux fluides sur tous les côtés, à l'exception de l'ouverture d'entrée (1a) et de l'ouverture d'évacuation (2a).

8. Dispositif de cathéter selon l'une des revendications 4, 5, 6 ou 7, **caractérisé en ce que** l'élément de fermeture (5, 13, 17) présente une membrane mobile (5) qui ferme de manière étanche aux fluides la chambre de commande de valve (12). et peut être déviée de telle sorte que l'ouverture d'entrée (1a) ou l'ouverture de sortie (2a) peut être fermée sélectivement par des parties de la membrane (5).

9. Dispositif de cathéter selon l'une des revendications 4 à 8, **caractérisé en ce qu'**un levier d'entraînement (3, 14) de l'entraînement de valve (3, 14, 18, A, A', B, B') dévie la membrane (5) au moins dans la première et la deuxième position.

10. Dispositif de cathéter selon l'une des revendications 4 à 9, **caractérisé en ce que** l'élément de fermeture (5, 13, 17) peut être entraîné par un entraînement de valve à action magnétique (A, A', B, B').

11. Dispositif de cathéter selon la revendication 9 ou 10, **caractérisé en ce que** l'élément de fermeture (5, 13, 17) disposé dans la chambre de commande de valve (12, 12a) est actif magnétiquement et interagit avec un champ magnétique de l'entraînement de valve (A, A', B, B').

12. Dispositif de cathéter selon la revendication 8 ou 9, **caractérisé en ce que** le levier d'entraînement (3, 14) peut être entraîné magnétiquement et/ou **en ce que** la chambre de commande de valve peut être séparée d'un entraînement de valve de l'élément de fermeture.

13. Dispositif de cathéter selon la revendication 11 ou 12, **caractérisé en ce que** tant la chambre de commande de valve (12, 12a) que les parties (3, 14, 18) de l'entraînement de valve qui sont reliées mécaniquement à l'élément de fermeture (5, 13, 17), à l'exception de l'ouverture d'entrée et de vidange (1a, 2a), sont fermées de manière étanche aux fluides et peut être séparée d'un dispositif générateur de champ magnétique (A, A', B, B') de l'entraînement de valve et /ou **en ce que** l'élément de fermeture est déplacé de préférence dans la troisième position (III) par un élément ressort élastique (10, 18).

14. Dispositif de cathéter selon l'une des revendications 4 à 13, **caractérisé en ce qu'**un aimant (13", 13‴, 13"") est prévu directement au niveau de la chambre de commande de valve (12, 12a), en particulier à l'intérieur de l'élément de fermeture (5, 13, 17), en tant que partie d'un dispositif de séparation ; dans lequel le ou les aimants (13", 13‴, 13"") sont prévus séparés, en particulier espacés, en particulier des ancrages d'entraînement (53, 54, 62, 63) de l'entraînement de valve.
